(19) Europäisches Patentamt
European Patent Office
Office européen des brevets

(11) Publication number: **0 082 731**
**A1**

(12) # EUROPEAN PATENT APPLICATION

(21) Application number: 82306879.6

(22) Date of filing: 22.12.82

(51) Int. Cl.³: C 07 C 103/52
A 61 K 37/28

(30) Priority: 23.12.81 DK 5763/81

(43) Date of publication of application:
29.06.83 Bulletin 83/26

(84) Designated Contracting States:
AT BE CH DE FR GB IT LI NL SE

(71) Applicant: NOVO INDUSTRI A/S
Novo Allé
DK-2880 Bagsvaerd(DK)

(72) Inventor: Jorgensen, Karin Damm
12, Skovvej
DK-2950 Vedbaek(DK)

(74) Representative: Brown, John David et al,
FORRESTER & BOEHMERT Widenmayerstrasse 4/I
D-8000 München 22(DE)

(54) **The use of peptides as medicaments.**

(57) Certain glucagon derivatives containing the glucagon moiety His-Ser-Gln-Gly-Thr-Phe-Thr-Ser-Asp-Tyr-Ser-Lys-Tyr-Leu-Asp-Ser-Arg-Arg-Ala-Gln-Asp- connected to hydroxy or one of the peptid chains -Phe-Val-Gln-Trp-Leu or -Met-Asn-Thr or one of the last two peptide chains with one or more of the amino acids therein left out, have an insulin releasing effect under hyperglycaemia conditions.

EP 0 082 731 A1

Croydon Printing Company Ltd.

Title: The Use of Peptides as Medicaments.

The present invention relates to the use of peptides of the general formula I

R'-R" (I)

wherein R' represents His-Ser-Gln-Gly-Thr-Phe-Thr-Ser-Asp-Tyr-Ser-Lys-Tyr-Leu-Asp-Ser-Arg-Arg-Ala-Gln-Asp-, and R" represents OH, the peptide chain -Phe-Val-Gln-Trp-Leu or -Met-Asn-Thr or a corresponding peptide chain or amino acid which is identical with one of the two last-mentioned peptide chains with the exception that one or more of the amino acid(s) has/have been omitted, or salts thereof as an insulin releasing agent.

As examples of the above "corresponding peptide chain or amino acid which is identical with one of the two last-mentioned peptide chains with the exception that one or more of the amino acid(s) has/have been omitted" -Val-Trp and -Met-Asn can be mentioned.

Glucagon, a polypeptide hormone consisting of 29 amino acids, is known to possess several pharmacological effects such as a spasmolytic effect on smooth muscle, an inhibitory effect on gastric acid secretion, and metabolic effects, for example, an insulin releasing effect.

It is known that glucagon releases insulin from the isolated perfused rat pancreas. It has now been found that glucagon(1-21) has no such effect when it is infused to the same concentration as glucagon, provided the buffer glucose concentration is 0.6 mg/ml corresponding to hypoglycaemia. Furthermore, it has now been found that glucagon-(1-21) - contrary to glucagon - does not cause hyperglycaemia or release insulin in vivo in normally fed rats. However, surprisingly, it has been found that glucagon(1-21) possesses about 85% of glucagon's IRI (immunoreactive insulin) releasing effect in the perfused rat pancreas model when the buffer glucose concentration is 2.0 mg/ml corresponding to hyperglycaemia, and about 60% of glucagon's IRI releasing effect when the buffer glucose concentration is 1.5 mg/ml corresponding to mild hyperglycaemia. Studies in glucose primed rats and pigs have now shown that glucagon-(1-21) infused intravenously improves the glucose tolerance, vide Experiments A to C below. As glucagon(1-21) possesses

glucagon's IRI releasing effect in the presence of hyperglycaemia yet at the same time being devoid of glucagon's glycogenolytic and glyconeogenetic effects, glucagon(1-21) may be used in the treatment of diabetics with remaining beta-cell function. Such a use should be especially safe as no hypoglycaemia will occur during treatment because of the lack of IRI releasing effect in the presence of hypoglycaemia and normoglycaemia. When glucagon(1-21) is infused to rats and pigs with plasma glucose levels below 1.2 mg/ml, glucagon(1-21) will neither release IRI nor influence the plasma glucose levels, vide Experiment D below.

In Diabetes 21 (1972), 843, it has been stated in the summary that the structure necessary for insulinogenic action is located inside the 24-29 amino acid sequence in glucagon. Contrary to this it has now, surprisingly, been found that the structure for insulinogenic action is in the 1-21 amino acid sequence, provided that hyperglycaemia is present.

Furthermore, it has, surprisingly, been found that glucagon(1-26) and des(22-26)glucagon possesses an insulin releasing effect in the perfused rat pancreas model when the buffer glucose concentration is 2.0 mg/ml.

It is to be expected that all compounds of formula I have an insulin releasing effect at hyperglycaemia because glucagon, glucagon(1-26), des(22-26)glucagon, and glucagon-(1-21) have this effect. However, contrary to glucagon(1-21), glucagon also has an insulin releasing effect in the presence of normoglycaemia and hypoglycaemia.

It has been stated in Danish patent application No. 2885/81 that compounds of formula I possess spasmolytic effect and an inhibitory effect on gastric acid secretion. Furthermore, it is stated therein that compounds of formula I show no or minor, negligible metabolic effects. The statement in said Danish application that glucagon(1-21) does not release insulin from the isolated perfused rat pancreas, applies if the buffer glucose concentration is 1.0 mg/ml

corresponding to normoglycaemia or below that concentration. Based thereupon it is surprising that compounds of formula I have an insulin releasing effect on animals primed with glucose corresponding to hyperglycaemia.

The metabolic effects of glucagon(1-21), glucagon-(1-26), and des(22-26)glucagon, as exemplified by their lipolytic effect on rat free fat cells in vitro and their effect on the activation of the adenylate cyclase in vitro, are negligible compared with the metabolic effects of glucagon. No metabolic effects have been found after administration to normally fasted and fed rats in vivo.

Hence, compounds of formula I or salts thereof may be used as an insulin releasing agent. Compounds of formula I or salts thereof are then useful for treatment of diabetics, for example, such with remaining beta-cell function. The fact that compounds of formula I also show spasmolytic effect and an inhibitory effect on the secretion of gastric acid will not, in many, if not most instances, preclude the use of the compounds of formula I as an insulin releasing agent. On the contrary the combination of the effects may prove to be an advantage, for example, in type 2 diabetics.

As examples of specific, known compounds of formula I glucagon(1-21), glucagon(1-23), glucagon(1-25), glucagon(1-26), and des(22-26)glucagon can be mentioned. The remaining compounds of formula I and salts thereof can be prepared by methods which, generally, are known in peptide synthesis and in this connection reference is, for example, made to Danish patent application No. 2885/81; Hoppe-Seyler's Z. Physiol.Chem. 362 (1981), 665 - 677; Methoden der Organischen Chemie (Houben-Weyl), Ed.: Müller, Vol. XV/1 + 2, G. Thieme Verlag, Stuttgart, 1974; The Peptides, Ed.: Gross and Meienhofer, Vol. 1 - 4, Academic Press, 1981; and Appl.Biochem.Biotech. 7 (1982), 385 et seq..

Compounds of formula I are converted into pharmaceutical preparations and administered, preferably to humans, in analogy with known methods.

Compounds of formula I and salts thereof may be administered intraveneously, intramuscularly or subcutaneously at dosages in the range of from about 1 to 1000 μg/kg body weight, preferably from about 10 to 100 μg/kg body weight, although a lower or higher dosage may be administered. The required dosage will depend on the severity of the condition of the patient and the number of administrations per day. Furthermore, compounds of formula I and salts thereof may be administered by the nasal or rectal route.

Compounds of formula I may possibly be administered orally, for example, by the use of special additives.

For the purpose of parenteral administration, compounds of formula I may be dissolved in distilled water and the pH-value is, if desired, adjusted with a suitable buffer so as to be in the range from about 6 to 8. Furthermore, the solution may be adjusted to isotonicity, for example, by the addition of about 0.9% sodium chloride. In order to facilitate the lyophilization process resulting in a suitable product, for example, lactose may be added to the solution. Thereafter, the solution may be sterile filtered and filled in vials. Thereafter, the solutions may be lyophilized and the vials may be sealed under aseptic conditions.

Other pharmaceutical methods may be employed to control the duration of action. Controlled release preparations may be achieved by the use of polymers to complex or absorb compounds of formula I. The controlled delivery may be excercised by selecting appropriate macromolecules (for example polyesters, polyamino acids, polyvinylpyrrolidone, ethylenevinylacetate, methylcellulose, carboxymethylcellulose, and protamine sulfat) and the concentration of macromolecules as well as the methods of incorporation in order to control the drug release. Besides, it is possible to

obtain a longer duration of action by parenteral administration of a suspension of the compounds in form of crystals or aggregates than by an aqueous solution because the compounds are continuously dissolving, a process which may be controlled by well known factors (for example crystal form, type of aggregates, and surface area). Another possible method to control the duration of action by controlled release preparations is to incorporate compounds of formula I into particles of a polymeric material in form of matrix or bioerodable systems, for example, glucagon(1-21) in polyesters, polyamino acids, hydrogels, poly(lactic acid) or ethylene vinylacetate copolymer. Instead of incorporating the compounds in polymeric particles it is possible to entrap compounds of formula I in microcapsules prepared, for example, by coacervation techniques or by interfacial polymerisation, for example, hydroxyethylcellulose or gelatine microcapsules and poly(methylmethacrylate) microcapsules, respectively, or in colloidal drug delivery systems, for example, liposomes, albumin microspheres, microemulsions, nanoparticles, and nanocapsules or in macroemulsions. Another mechanism to achieve retarded preparations is through the use of biological acceptable oil solutions (for example viscoleo and arachis oil) where the release of drug is controlled by partitioning of drug out of the oil into the surrounding aqueous milieu. Besides it is possible to use an oil suspension which combines the principles involved in aqueous suspensions and oil solutions.

For the purpose of nasal administration a solution in a nasal spraying device or nebulisator is used. The compounds of formula I are dissolved in distilled water and the pH-value is adjusted so as to be in the range from about 6 to 8, for example, by adding sodium phosphate and citric acid as buffer. Sodium chloride, sorbitol and glycerol may be used to obtain an isotonic solution with a suitable viscosity. The solution may be administered by the use of a

suitable nebulisator or plastic spray. The solution may be preserved by the use of known preservatives, for example, methyl or propyl p-hydroxybenzoate. In order to promote the efficacy, a surfactant, for example, esters of polyoxyethylene, fatty acids or enamine derivatives, for example, N-(1-methyl-2-ethoxycarbonylvinyl)-D-phenylglycine, may serve as examples for adsorption accelerators. For the purpose of nasal administration by the use of dose aerosol spray a compound of formula I is mixed with suitable constituents and a mixture of halogencarbons, for example, monofluorotrichloromethane, difluorodichloromethane, and tetrafluorodichloroethane, in order to obtain a mixture with a vapour pressure producing a well defined single dose when the mixture is administered by the use of a dose aerosol spray.

By nasal administration the compounds of formula I are preferably used in a dosage range between about 0.1 and 100 µg/kg body weight, preferably between 1 and 10 µg/kg body weight, per single dose. This dose could be administered several times per day.

For the purpose of rectal administration suppositories are produced by admixing a compound of formula I with an inactive constituent such as cocoa butter or with a base such as Polysorbate 85, propylene glycol monostearate and white bee's wax. Surfactants and/or enamine derivatives may serve as examples of adsorption promotors.

A preferred subclass of compounds of formula I is compounds wherein the amino acid sequence is identical with a continuous part of the amino acid sequence of glucagon. As examples of specific compounds of formula I compounds wherein R" is Phe, Val, Gln, Trp, Leu, Met, Asn or Thr, can be mentioned. A preferred compound of formula I is glucagon(1-21) because it shows superior pharmacological properties and because it can easily be obtained, for example, from natural glucagon.

As examples of salts of compounds of formula I sodium, potassium, magnesium, calcium, and zinc salts and acid addition salts with organic or inorganic acids such as formic acid can be mentioned. Preferred salts of compounds of formula I are physiologically and pharmaceutically acceptable salts.

The present invention also relates to a pharmaceutical composition comprising a compound of formula I or a salt thereof and one or more pharmaceutically acceptable carrier(s), diluent(s), preferably water, and/or exipient(s) when used as an insulin releasing medicament. As examples of such carriers conventional preservatives, for example, methyl or propyl p-hydroxybenzoate, and sodium chloride can be mentioned.

Any novel feature or combination of features described herein is considered essential.

The three-letter abbreviations for amino acids used herein are stated in J.Biol.Chem. 243 (1968), 3558. For the sake of brevity, glucagon-(1-21)-heneicosapeptide herein has been designated glucagon(1-21) and, analogously, this applies for glucagon(1-23), glucagon(1-25), and glucagon(1-26). Furthermore, des-pentapeptide-(22-26)-glucagon has been designated des(22-26)glucagon.

The following example which, however, is not considered to be limiting, is presented to illustrate the invention.

Example

A preparation for parenteral administration containing 1 mg of glucagon(1-21) per ml may be prepared as follows:

1 g of glucagon(1-21) and 99 g of lactose are dissolved in 1 litre of distilled water and the pH-value is adjusted to 7.0. The solution is thereafter sterile

filtered. The sterile solution is filled in 10 ml vials in such a way that each vial contains 1.0 ml of the solution. Thereafter, the solutions are lyophilized and the vials are sealed under aseptic conditions.

The preparation in any of the vials is to be dissolved in 1.0 ml of sterile water before administration.

Further examples of preparing preparations containing glucagon(1-21) are stated in Danish patent application No. 2885/81. Preparations containing other compounds of formula I are prepared analogously.

EXPERIMENT A:

Effect on IRI-Release in Rats **in vivo**.

10 male rats, Wistar strain, 150 g (+/- 5 g) were anaesthetized with Pentobarbital. Polyethylene catheters were inserted into the right carotid artery for blood sampling and the left jugular vein for infusions. The rats were divided into two groups with 5 animals in each group. One group was infused with glucose (1.5 mg/min) from 0 to 60 minutes (placebo) and one group was infused with glucose (1.5 mg/min) + glucagon(1-21) (100 µg/kg/h) from 0 to 60 minutes. Blood was sampled into glasses on ice containing heparin (250 IU/ml) and aprotinin (600 KIU/ml) at the times 0, 1, 10, 20, 30, 40, 50, and 60 minutes. Insulin was determined by radioimmuno assay (RIA) on plasma. The results are stated in Table I below.

Table I

| | IRI, µU/ml. | | | | | | | |
|---|---|---|---|---|---|---|---|---|
| Placebo | 7 | 20 | 32 | 40 | 40 | 40 | 34 | 36 |
| Glucagon(1-21) | 18 | 43 | 48 | 62 | 56 | 56 | 60 | 55 |
| Time, min | 0 | 1 | 10 | 20 | 30 | 40 | 50 | 60 |

EXPERIMENT B:

Effect on Plasma Glucose Levels in Rats in vivo.

1. Intravenous Administration.

Male Wistar rats were prepared as described in Experiment A. One group of 5 rats had an intravenous infusion of glucose (2 mg/min) from 0 to 30 minutes followed by infusion of 0.9% saline with 0.1% HSA (human serum albumin). One group of 5 rats had an intraveneous infusion of glucose (2 mg/min) from 0 to 30 minutes followed by an infusion of 1 mg of glucagon(1-21) in saline with HSA from 30 to 60 minutes. Blood samples were drawn from the carotid artery at the times 0, 2, 5, 10, 20, 25, 30, 40, 50, and 60 minutes and blood glucose values were determined by the hexokinase method on an autoanalyzer. The blood glucose values are stated in Table II below.

Table II

| | Blood glucose, mg/ml | | | | | | | | | |
|---|---|---|---|---|---|---|---|---|---|---|
| Placebo | 0.9 | 1.1 | 1.3 | 1.4 | 1.7 | 1.8 | 1.8 | 1.5 | 1.3 | 1.2 |
| Glucagon-(1-21) | 1.0 | 1.2 | 1.4 | 1.5 | 1.7 | 1.9 | 1.9 | 1.3 | 1.0 | 0.8 |
| Time, min | 0 | 2 | 5 | 10 | 20 | 25 | 30 | 40 | 50 | 60 |

2. Peroral Administration of Glucose.

The effect of glucagon(1-21) was investigated in rats during an oral glucose tolerance test. 20 male Wistar rats were divided into 2 groups of 10 rats and dosed as follows: Both groups were dosed with glucose (5 g/kg, perorally) at 0 and 60 minutes. The placebo group was dosed with 1 ml of 0.9% saline with 0.1% HSA intraperitoneally at the times 0, 30, 60, and 90 minutes. The other group was

dosed with glucagon(1-21) (1 mg/kg) in 0.9% saline with HSA at the times 0, 30, 60, and 90 minutes. Blood samples were drawn from the tails at the times -5, 0, 15, 30, 45, 60, 90, and 120 minutes for blood glucose determination. The blood glucose values are stated in Table III below.

Table III

| | Blood glucose, mg/ml | | | | | | | |
|---|---|---|---|---|---|---|---|---|
| Placebo | 0.88 | 0.98 | 1.60 | 1.52 | 1.46 | 1.45 | 1.60 | 1.54 |
| Glucagon-(1-21) | 0.89 | 0.96 | 1.32 | 1.35 | 1.23 | 1.36 | 1.37 | 1.37 |
| Time, min | -5 | 0 | 15 | 30 | 45 | 60 | 90 | 120 |

EXPERIMENT C:

Effect on Blood Glucose Levels in Pigs in vivo.

1. Intravenous Administration.

The effect of glucagon(1-21) on plasma glucose levels was investigated during an intravenous glucose infusion. 4 female pigs (Danish breed/Yorkshire) weighing 30 kg (+/- 3 kg) were dosed with glucose (0.7 g/kg/h) as an intravenous infusion and with the same glucose infusion + infusion of glucagon(1-21) (100μg/kg/h) from 0 to 120 minutes. The experiment was carried out as a cross-over experiment. The pigs were infused through intravenous ear catheters and blood samples were taken from the other ear at the times 0, 15, 30, 45, 60, 75, 90, 105, and 120 minutes. Glucose was analyzed by the hexokinase method and the blood glucose values are stated in Table IV below.

Table IV

| | Plasma glucose, mg/ml | | | | | | | | |
|---|---|---|---|---|---|---|---|---|---|
| Placebo | 1.0 | 1.7 | 2.1 | 2.2 | 2.3 | 2.3 | 2.3 | 2.2 | 2.3 |
| Glucagon-(1-21) + glucose | 0.9 | 1.5 | 1.7 | 1.8 | 1.9 | 1.8 | 2.0 | 1.9 | 1.9 |
| Time, min | 0 | 15 | 30 | 45 | 60 | 75 | 90 | 105 | 120 |

2. Peroral Administration of Glucose.

The effect of glucagon(1-21) on plasma glucose levels was investigated during an oral glucose tolerance test in pigs. 4 pigs were dosed in a cross-over experiment with glucose (5 g/kg) perorally at 0 and 60 minutes + placebo (saline with HSA) or glucagon(1-21) (1 mg/kg/h) from 0 to 120 minutes intravenously. Blood samples were drawn from an ear vein at the times -5, 10, 20, 30, 45, 60, 75, 90, 105, and 120 minutes and plasma glucose was assayed by the hexokinase method. The plasma glucose values are stated in Table V below:

Table V

| | Plasma glucose, mg/ml. | | | | | | | | | |
|---|---|---|---|---|---|---|---|---|---|---|
| Placebo | 0.8 | 1.3 | 1.8 | 2.2 | 2.5 | 2.5 | 2.7 | 2.8 | 2.7 | 2.4 |
| Glucagon-(1-21) | 0.8 | 1.0 | 1.4 | 1.7 | 1.8 | 1.9 | 1.8 | 1.7 | 1.7 | 2.1 |
| Time, min | -5 | 10 | 20 | 30 | 45 | 60 | 75 | 90 | 105 | 120 |

EXPERIMENT D:

Studies of the Effect of Glucagon and Glucagon(1-21) on Plasma Glucose and Plasma IRI Levels in Normoglycaemic Rats.

Glucagon (1 mg/kg) and an equimolar dosis of glucagon(1-21), i.e. 0.77 mg/kg, were injected intravenously at the beginning time (time: 0 minutes) to normally fed male Wistar rats weighing 150 g (+/- 5 g). Blood samples were taken from the orbital plexus at the times -5, 2, 5, 10, 15, 30, and 60 minutes. Blood glucose was assayed according to the hexokinase method and plasma insulin was assayed by RIA. Glucagon had a significantly increasing effect on blood glucose and plasma IRI. Contrary to glucagon, glucagon(1-21) had no effect on these parameters. Mean blood glucose values (N = 10) are stated in Table VI below, and mean plasma IRI values (N = 10) are stated in Table VII below.

Tabel VI

| | Blood glucose, mg/ml | | | | | | | |
|---|---|---|---|---|---|---|---|---|
| Glucagon | 0.9 | 1.2 | 1.3 | 1.3 | 1.4 | 1.5 | 1.1 | experimental |
| Placebo | 0.9 | 1.1 | 1.1 | 1.2 | 1.2 | 1.1 | 1.3 | day 1 |
| Glucagon(1-21) | 1.0 | 1.1 | 1.1 | 1.2 | 1.3 | 1.3 | 1.3 | experimental |
| Placebo | 1.0 | 1.2 | 1.2 | 1.3 | 1.3 | 1.3 | 1.3 | day 2 |
| Time, min | -5 | 2 | 5 | 10 | 15 | 30 | 60 | |

Table VII

| | Plasma IRI μU/ml | | | | | | | |
|---|---|---|---|---|---|---|---|---|
| Glucagon | 19 | 95 | 98 | 82 | 67 | 53 | 17 | experimental |
| Placebo | 17 | 25 | 28 | 23 | 20 | 22 | 25 | day 1 |
| Glucagon(1-21) | 32 | 30 | 33 | 35 | 43 | 38 | 30 | experimental |
| Placebo | 13 | 20 | 20 | 22 | 24 | 22 | 16 | day 2 |
| Time, min | -5 | 2 | 5 | 10 | 15 | 30 | 60 | |

EXPERIMENT E:

Acute Toxicity Study

10 mg glucagon(1-21) administered intravenously as a bolus to NMRI mice weighing about 20 g (i.e. a dose of about 500 mg/kg body weight) had no adverse effects. No deaths occurred.

CLAIMS

1. The use, as an insulin releasing agent, of compounds of the general formula I

R'-R" (I)

wherein R' represents His-Ser-Gln-Gly-Thr-Phe-Thr-Ser-Asp-Tyr-Ser-Lys-Tyr-Leu-Asp-Ser-Arg-Arg-Ala-Gln-Asp-, and R" represents hydroxy, the peptide chain -Phe-Val-Gln-Trp-Leu or -Met-Asn-Thr or a corresponding peptide chain or amino acid which is identical with one of the two last-mentioned peptide chains with the exception that one or more of the amino acid(s) has/have been omitted, or salts thereof.

2. The use, according to Claim 1, wherein the amino acid sequence in the compound of formula I is identical with the sequence in a fragment of glucagon.

3. The use, according to Claim 1, wherein R" represents Phe, Val, Gln, Trp, Leu, Met, Asn or Thr.

4. The use, according to Claim 1, wherein R" represents hydroxy, -Phe-Val-Gln-Trp-Leu or -Met-Asn-Thr.

5. The use, according to any of the Claims 1, 2, and 4, wherein R" represents hydroxy.

6. The use, according to any of the preceding claims, characterized in that the compound of formula I is used for treatment of diabetics.

7. The use, according to Claim 6, characterized in that the compound of formula I is used for treatment of diabetics with remaining beta-cell function.

8. The use, according to any of the preceding claims, characterized in that the compound of formula I is used in an effective amount.

9. A pharmaceutical composition which comprises an effective amount of a compound of formula I, according to Claim 1, or a salt thereof in association with a suitable physiologically acceptable carrier, diluent and/or excipient when used as an insulin releasing medicament.

10. A pharmaceutical composition, according to Claim 9, characterized in that it comprises between 7.5 and 75,000 μg, preferably between 75 and 7500 μg, of a compound of formula I or a salt thereof per dosage unit.

11. A pharmaceutical composition, according to Claim 9 or 10, characterized in that it contains a compound of formula I, wherein the amino acid sequence is identical with the sequence in a fragment of glucagon.

12. A pharmaceutical composition, according to Claim 9 or 10, characterized in that it contains a compound of formula I, wherein R" represents Phe, Val, Gln, Trp, Leu, Met, Asn or Thr.

13. A pharmaceutical composition, according to Claim 9 or 10, characterized in that it contains a compound of formula I, wherein R" represents hydroxy, -Phe-Val-Gln-Trp-Leu or -Met-Asn-Thr.

14. A pharmaceutical composition, according to Claim 9, 10 or 13, characterized in that it contains a compound of formula I, wherein R" is hydroxy.

15. The use of compounds of formula I stated in Claim 1, preferably the compounds used according to any of the Claims 2 to 5, or a salt thereof for the preparation of medicaments having an insulin releasing effect.

16. A compound of formula I stated in Claim 1, preferably the compounds used according to any of the Claims 2 to 5, for use as an insulin releasing agent.

17. An insulin releasing agent characterized in that it comprises a compound of formula I stated in Claim 1, preferably the compounds used according to any of the Claims 2 to 5.

18. The use of a compound of formula I stated in Claim 1, preferably the compounds used according to any of the Claims 2 to 5, for treating persons normally having too low a production of insulin.

19. The method for treating or preventing hyperglycaemia characterized by administering for insulin release purposes a compound of formula I stated in Claim 1, preferably the compounds used according to any of the Claims 2 to 5.

0082731

European Patent Office

# EUROPEAN SEARCH REPORT

Application number

EP 82 30 6879

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | THE JOURNAL OF BIOLOGICAL CHEMISTRY, vol. 247, no. 4, 25th February 1972, pages 1211-1218, (USA); I.D.GOLDFINE et al.: "Glucagon receptors in beta-cells. Binding of 125I-glucagon and activation of adenylate cyclase". *The whole document* | 1-6,9-16 | C 07 C 103/52<br>A 61 K 37/28 |
| | --- | | |
| P,X | EP-A-0 044 168 (NOVO) *The whole document* | 1-19 | |
| | ----- | | |
| | | | TECHNICAL FIELDS SEARCHED (Int. Cl. 3) |
| | | | C 07 C 103/00<br>A 61 K 37/00 |

The present search report has been drawn up for all claims

| Place of search | Date of completion of the search | Examiner |
|---|---|---|
| THE HAGUE | 18-03-1983 | RAJIC M. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503. 03.82